# EUROPÄISCHE PATENTSCHRIFT

(11) **EP 2 929 879 B1**
(45) Veröffentlichungstag und Bekanntmachung des Hinweises auf die Patenterteilung: **18.03.2020**
(21) Anmeldenummer: 15162540.7
(22) Anmeldetag: 07.04.2015
(51) Int. Cl.: A61K 31/122, A61K 31/197, A61K 31/355, A61K 31/375, A61K 31/4188, A61K 31/4415, A61K 31/51, A61K 31/519, A61K 31/525, A61K 31/593, A61K 31/714, A61P 43/00

(54) **KOMBINATIONSPRÄPARAT GEGEN INFERTILITÄT, DIE MIT ENDOMETRIOSE ASSOZIIERT IST**
COMBINATION PREPARATION FOR TREATING INFERTILITY WHICH IS ASSOCIATED TO ENDOMETRIOSIS
PRÉPARATION COMBINÉE POUR LE TRAITEMENT DE L'INFERTILITÉ ASSOCIEÉ AVEC L'ENDOMETRIOSE

(30) Priorität: 07.04.2014 AT 502622014
(43) Veröffentlichungstag der Anmeldung: 14.10.2015
(73) Patentinhaber: Gonadosan GmbH, 9244 Niederuzwil (CH)
(72) Erfinder: Wogatzky, Birgit, 88131 Lindau (DE); Wogatzky, Johannes, 88131 Lindau (DE)
(74) Vertreter: Burger, Hannes

(56) Entgegenhaltungen:
- WO-A1-94/17799
- WO-A1-2004/052351
- WO-A1-2012/076680
- AT-A1- 510 761
- AU-A4- 2008 100 822

## Beschreibung

Die Erfindung betrifft ein Kombinationspräparat zur Verwendung in der Behandlung Endometriose induzierter weiblicher Sub- und Infertilität umfassend zumindest die Wirkstoffe C-Vitamin, E-Vitamine, B1-Vitamin, B2-Vitamin, B5-Vitamin, B6-Vitamine, B7-Vitamin, B9-Vitamin, B12-Vitamine, gegebenenfalls D-Vitamine, Niacin, Zink, Eisen, Selen, Jod, Kupfer, N-Acetylcystein, Lycopen, Omega-3-Fettsäuren und Ubiquinon-10 sowie dessen Verwendung.

Immer mehr Paaren ist der Weg zum Wunschkind erschwert. In Industrieländern wie Deutschland und USA ist heute bereits eines von zehn Paaren ungewollt kinderlos und die Tendenz ist steigend.

Neben medizinischen Ursachen ist auch die Bedeutung einer ausgeglichenen Lebensführung mit einer gesunden und ausgewogenen Ernährung für die Fruchtbarkeit bei Mann und Frau unbestritten. Eine gute Versorgung mit Vitaminen und gewissen Nährstoffen fällt jedoch auch bei besten Absichten manchmal schwer. Bei erhöhten Belastungen im Alltag mit viel Stress und ungesunder Ernährung kommt der Körper täglich zu kurz. Infolge dessen können Immunsystem, die körperliche Homöostase und der Zellstoffwechsel entgleisen. Das betrifft speziell die Zellen, die besonders empfindlich sind. Dazu gehören insbesondere auch die Ei- und Samenzellen. Eizellen gelten als besonders sensibel, da sie bereits von Geburt an im Körper des Mädchens angelegt sind und bei unzureichender Nährstoffzufuhr oder erhöhten Belastungen bereits jahrelange Belastungen hinter sich haben, bevor sie dann, zum Zeitpunkt des Kinderwunsches der gereiften Frau, mit hoher Teilungsrate und Zellaktivität entscheidend zu dem Erfolg einer Schwangerschaft beitragen müssen. Samenzellen werden hingegen fortwährend in einem 90 tägigen Zyklus vom Körper neu gebildet, haben jedoch durch ihr fehlendes Zytoplasma nur wenige Reparatur- und Schutzenzyme zur Verfügung. Die männlichen Spermien werden daher durch Zell-Stoffwechsel-Schäden zunehmend träge (Asthenozoospermie), werden weniger (Oligozoospermie) und/ oder verändern ihre Form (Teratozoospermie). Das Vollbild einer Spermienschädigung durch veränderten Stoffwechsel oder Zellschäden nennt sich daher Oligo-Astheno-Teratoszoospermie. Auch die im Spermienköpfchen transportierte Erbsubstanz kann Schaden nehmen (fragmentieren), dadurch ist die Fähigkeit, eine Eizelle zu befruchten, deutlich reduziert. Eizellen werden in der Reifung durch einen veränderten Stoffwechsel ebenfalls gehemmt und der Eisprung kann ausbleiben (Anovulation) oder der verbliebene Gelbkörper ist geschwächt (Lutealinsuffizienz). Durch all diese Einflüsse kann die Fruchtbarkeit eines Paares unter einer Fehl- oder Mangelernährung leiden. Diese und noch viele weitere Folgen schlechter Ernährung sind inzwischen ausreichend untersucht worden und unterstreichen, wie sehr die richtige Ernährung zur Erfüllung des Kinderwunsches beitragen kann.

Experten empfehlen, den weiblichen Körper bereits einige Monate vor einer geplanten Schwangerschaft über Nahrungsergänzungsmittel zusätzlich mit wichtigen Vitalstoffen und Mineralien zu versorgen. Vor und während einer Schwangerschaft sowohl gesunder Frauen als auch Frauen mit spezifischen Erkrankungen ist eine ausreichende und dem jeweiligen Stadium entsprechende Versorgung mit essentiellen Nährstoffen und Vitaminen für die Gesundheit des heranwachsenden Babys förderlich. Allerdings kann sich ein übermäßiger regelloser Vitaminkonsum in einigen Fällen sogar schädlich auf die Eizellqualität auswirken.

Die AT 510 761 A1 beschreibt ein Kombinationspräparat umfassend zumindest die Wirkstoffe C-Vitamin, E-Vitamine, B1-Vitamin, B2-Vitamin, B5-Vitamin, B6-Vitamine, B9-Vitamin, B12-Vitamine, D-Vitamine, zumindest eine Ubiquinon-10-Komponente, und B7-Vitamin in der Behandlung von weiblicher Infertilität sowie zur Verbesserung der Eizellqualität und Eizellreife.

Die WO 2012/076680 A1 beschreibt ein Kombinationspräparat zur Behandlung weiblicher Infertilität unter anderem von Hashimoto-Patientinnen. Das Kombinationspräparat enthält zumindest die Wirkstoffe C-Vitamin, E-Vitamine, B1-Vitamin, B2-Vitamin, B3-Vitamin, B5-Vitamin, B6-Vitamine, B7-Vitamin, B9-Vitamin, B12-Vitamine, D-Vitamine, Zink, Magnesium, Eisen, Selen und/oder eine Selenverbindung, und Ubiquinon-10.

Die AU 2008/100822 A4 beschreibt ebenfalls ein Nahrungsergänzungsmittel zur Behandlung weiblicher Infertilität bestehend aus einer Tablette enthaltend die Wirkstoffe Ascorbinsäure, Thiamin, Riboflavin, Kalzium-Panthothenat, Pyridoxin HCl, Folsäure, B 12-Vitamin und Biotin sowie Nicotinamid, Selen, Mangan, Zink, Magnesium, Eisen, Jod, Kupfer sowie eine Kapsel enthaltend E-Vitamin, C-Vitamin, Coenzym Q10, sowie Betacarotin und Omega-3-Fettsäuren, wobei das Nahrungsergänzungsmittel für die Förderung der Empfängnis bei Frauen, die über einen Zeitraum von 6 bis 12 Monaten nicht schwanger werden konnten, eingesetzt wird und dabei die Tablette und die Kapsel gemeinsam eingenommen werden.

Es gilt zu beachten, dass Frauen mit spezifischen Erkrankungen, die einen Kinderwunsch tragen, zudem besondere Bedürfnisse aufweisen. Dies gilt in besonderem Maße auch für Frauen mit Endometriose. Hierbei muss die Empfehlung gegeben werden, dass Endometriosepatientinnen aufgrund besonderer Erfordernisse ihrer zugrunde liegenden Erkrankung spezielle Präparate verwenden sollten, die den besonderen Bedürfnissen Rechnung tragen.

Endometriose ist eine häufige Erkrankung unter der ca. 25 % bis 30% der Frauen mit unerfülltem Kinderwunsch leiden. Es handelt sich dabei um Gebärmutterschleimhaut (lat. Endometrium) die an atypischer Stelle, z.B. im Bauchraum, an den Ovarien oder an anderen Bereichen des Körpers (z.B. Darm etc.) lokalisiert ist. Wie das Endometriumgewebe in diese anderen Körperregionen gelangt, ist allerdings bisher nicht abschließend geklärt. Was aber als sicher angenommen werden kann ist die Tatsache, dass diese an atypischer Stelle sitzenden Endometriose-Herde einen dauerhaften Entzündungsherd während der fertilen Phase der Frau darstellen können. Eine Endometriose beeinträchtigt die Fruchtbarkeit von Frauen zudem auf mehreren weiteren Ebenen.

Aufgabe vorliegender Erfindung ist es ein Kombinationspräparat zur Verfügung zu stellen, welches die Fertilität von Frauen mit Endometriose erhöht.

Die Aufgabe der Erfindung wird durch ein Kombinationspräparat umfassend zumindest die Wirkstoffe C-Vitamin, E-Vitamine, B1-Vitamin, B2-Vitamin, B5-Vitamin, B6-Vitamine, B7-Vitamin, B9-Vitamin, B12-Vitamine, gegebenenfalls D-Vitamine, Niacin, Zink, Magnesium, Eisen, Selen, Jod, Kupfer, N-Acetylcystein, Lycopen, Omega-3-Fettsäuren und Ubiquinon-10, wobei Lycopen in einer Menge mit einer Untergrenze von 8 mg im Kombinationspräparat enthalten ist, zur Verwendung in der Behandlung Endometriose assoziierter bzw. induzierter weiblicher Sub- bzw. Infertilität gelöst. Beim erfindungsgemäßen Kombinationspräparat ist von Vorteil, dass die spezielle Kombination der ausgewählten Wirkstoffe bessere Ergebnisse als im Vergleich zu den Einzelsubstanzen zeigt.

Viele Mikronutrienten bilden die Basis für eine Empfängnis sowie normale Schwangerschaft. Es werden durch gewisse Nährstoffe folgende Grundfunktionen der Fertilität unterstützt: eine ausgeglichene Hormonfunktion, ein funktionierendes Immunsystem, ein ausreichendes antioxidatives Schutzsystem und eine gute Blut- und Energieversorgung. Für einen ausgeglichenen Hormonhaushalt sorgen unteranderem Vitamin B5, Vitamin B6, Zink und Jod. Die Vitamine C, B6, B12, D Folsäure, Eisen und Zink sind an der Funktion des Immunsystems beteiligt.

Antioxidative Wirkung zeigen unter anderem Vitamin C, E, B2 und Zink. Für eine ausreichende Blutversorgung sind Vitamin B1, Vitamin B2, Vitamin B6, Vitamin B12, Folsäure und Eisen verantwortlich und für eine ausreichende Energieversorgung sorgen Vitamin C, Vitamin B1, Vitamin B2, Vitamin B6, Vitamin B12, Pantothensäure, Niacin, Biotin, Eisen, Magnesium, Jod und Coenzyme Q10.

Jeder dieser Bereiche ist bei Patientinnen mit Endometriose unter einer besonderen Belastungssituation. Daher kann die bedarfsgerechte Versorgung mit bestimmten Mikronährstoffen gerade bei Endometriosepatientinnen eine gesunde Empfängnis und Schwangerschaft ernährungsphysiologisch unterstützen. Vorteilhaft dabei erweist sich, dass durch das erfindungsgemäße Kombinationspräparat die Behandlung von Endometriose induzierter Sub- bzw. Infertilität über mehrere Ansatzpunkte auf unterschiedlichen Wegen ermöglicht wird.

Beginnend beim Hormonsystem der Endometriosepatientin zeigt sich, dass Endometriosepatientinnen ein anderes Hormonprofil im Vergleich zu gesunden Frauen aufweisen. Sie zeigen beispielsweise einen höheren Östrogen- und niedrigeren Progesteronspiegel.

Daneben spielt bei der Endometriose eine cytokinmediierte Entzündung eine entscheidende Rolle. Die Entzündung selbst wirkt sich zum einen direkt, zum anderen auch über mehrere Wirkmechanismen indirekt negativ auf die Empfängnis aus. Unter anderem bewirkt die Entzündungsreaktion eine Erhöhung der reaktiven Sauerstoffspezies und führt somit zu erhöhtem oxidativen Stress im weiblichen Reproduktionstrakt. Dies beeinträchtigt die sich entwickelnde, heranreifende Eizelle negativ und führt zu einer reduzierten Eizellqualität. Die reaktiven Sauerstoffspezies und entzündungsassoziierten Cytokine haben aber auch einen Einfluss auf die Befruchtungsfähigkeit der Spermien und auf die anschließende Embryonalentwicklung. Abnormale Ereignisse im Zellkern und Cytoplasma sind bei Embryonen von Endometriosepatientinnen sechsmal häufiger als bei Frauen ohne Endometriose. Schließlich können reaktive Sauerstoffspezies auch noch mitochondriale Schäden und DNA Strangbrüche in den Eizellen auslösen. Dies kann schlimmstenfalls programmierten Zelltod (Apoptose) verursachen.

Die modernen Techniken der assistierten Reproduktion, z.B die In-vitro-Fertilisation können die Infertilität von Endometriosepatientinnen glücklicherweise teilweise korrigieren. Allerdings liegen bezüglich der Erfolgsquote hier sehr unterschiedliche Daten vor. Es gibt mehrere Studien, die auch nach In-vitro-Fertilisation den Endometriosepatientinnen eine schlechtere Erfolgs-Prognose bescheinigen als Frauen ohne Endometriose.

Mit dem erfindungsgemäßen Kombinationspräparat wird die Erfolgsrate der Fertilitätsbehandlung gesteigert, weil gleichzeitig an unterschiedlichsten Stellen der Ursachen bzw. Auswirkungen von Endometriose angegriffen wird. Besonderes Augenmerk richtet das Kombinationspräparat dabei auf die ernährungsphysiologische Behandlung von Entzündungen und auf die Reduktion des mit der Entzündung zusammenhängenden oxidativen Stress, welcher, wie oben beschrieben, eine der Hauptursachen für Endometriose assoziierte Infertilität darstellt.

Oxidativer Stress kann zudem auch den Endometriose- bedingten Entzündungsprozess weiter anfachen. Bei Endometriosepatientinnen ist der antioxidative Schutz in der Peritoneal- und auch Follikelflüssigkeit vermindert, was sich auch negativ auf eine eventuell bereits begonnene Embryonalentwicklung auswirken kann. Dies und die Tatsache, dass eine Kinderwunschbehandlung oder Schwangerschaft, z.B. durch die hormonelle Stimulation, einen weiteren Stressor auf die Homöostase der Patientin ausübt, erklärt die Tatsache, dass insbesondere bei einer in-vitro-Fertilisation oder in der Schwangerschaft die ausreichende Versorgung mit Antioxidantien extrem wichtig ist.

Oxidativer Stress, wie er beispielsweise durch einen Mangel an Antioxidantien oder bei Reduktion von antioxidativen Enzymen durch Substratmangel begünstigt werden kann, beeinträchtigt die weibliche Fertilität auf verschiedene Weise. Durch oxidativen Stress wird der Alterungsprozess vorangetrieben und es kommt zu Fehlfunktionen, die zu einem Mangel an mitochondrialer Energiebereitstellung in den Eizellen führt. Dieser Energiemangel reduziert die Fähigkeit der Eizelle zur Befruchtung und vermindert zudem die Eizellqualität. Die durch Cytokine, TNF, Interleukine, etc. vermittelten Entzündungsreaktionen resultieren im Extremfall im Zelltod und Apoptose. Dies führt zur weiteren Freisetzung der Entzündungsmediatoren und im Sinne eines "circulus vitiosus" zur weiteren Erhöhung des oxidativen Stresses. Somit verschlechtert sich die Eizellsituation bei Endometriosepatientinnen zusehends.

Durch die Wirkstoffe C-Vitamin, E-Vitamine, verschiedene B-Vitamine, Folsäure und N-Acetyl-L-Cystein findet eine antioxidative Behandlung von diesem schädlichen oxidativem Stress statt.

C-Vitamin trägt zur normalen Funktion des Immunsystems sowie zum Schutz der Zellen vor oxidativem Stress bei. Es spielt zudem eine wichtige Rolle beim Eisenstoffwechsel. Durch die ausgiebige Versorgung mit C-Vitamin in Depotform kann ein Anstieg von antioxidativen Markern in Frauen mit Endometriose beobachtet werden. Zudem können durch die antioxidativen Wirkstoffe chronische Unterleibsschmerzen sowie Entzündungsmarker in der Peritonealflüssigkeit reduziert werden.

Auch E-Vitamine tragen zur antioxidativen Behandlung sowie zur Steigerung der Fertilität bei. Allerdings zeigen Studien, dass eine zu hohe Dosierung von E-Vitaminen zu einem reduzierten Geburtsgewicht führen kann. Daher werden E-Vitamine in moderater Dosierung eingesetzt.

Folsäure, B6-Vitamin und B12-Vitamin reduzieren den Homocysteinspiegel, welcher bei Endometriosepatientinnen häufig erhöht ist. Homocystein ist eine natürlich vorkommende (nicht proteinogene) α-Aminosäure. Sie entsteht im Stoffwechsel durch S-Demethylierung von L-Methionin als Methyldonor. Erhöhte Blutwerte für Homocystein können eine Schädigung der Blutgefäße zur Folge haben. Die angemessene Versorgung mit den Vitaminen B6, B12 und Folsäure wirkt sich positiv auf die Gefäßfunktion aus und unterstützt somit eine ausreichende Durchblutung der Eierstöcke. Auch die Implantation des Embryos in der Frühschwangerschaft wird durch einen ausgeglichenen Homocystein-Stoffwechsel positiv beeinflusst.

Die B-Vitamine umfassen 8 Vitamingruppen, B1 (Thiamin), B2 (Riboflavin), B3 (Nikotinsäure), B5 (Pantothensäure), B6 (Pyridoxin), B7 (Biotin), B9 (Folsäure), B12 (Cobalamin). Alle B-Vitamine spielen eine zentrale Rolle im Stoffwechselgeschehen und sind daher für eine gesunde Fortpflanzung unerlässlich. Biotinmangel beeinträchtigt die Fruchtbarkeit und führt zu einer erhöhten Fehlbildungsrate. Die besondere Bedeutung der Versorgung der zukünftigen Mutter mit Folsäure zum Schutz vor Spina bifida des Kindes ist seit Jahren bestens bekannt.

Die Spurenelemente Kupfer, Zink und Selen unterstützen die antioxidative Enzymaktivität, wodurch eine reduzierte antioxidative Enzymaktivität ausgeglichen wird. Selen ist ein Bestandteil der antioxidativen Enzyme Glutathionperoxidase und Thioredoxinreduktase, welche indirekt bestimmte oxidierte Moleküle reduzieren. Selen fungiert somit auch als wirksames Antioxidans. Bei Endometriosepatientinnen ist der Selenspiegel oft reduziert.

Kupfer und Zink sind wichtige Cofaktoren für das antioxidative System, indem sie für eine gute Funktion von verschiedenen antioxidativen Enzymen wie Katalase, Superoxiddismutase, Glutathionreduktase, Glutathionperoxidase und Molekülen wie Glutathion und NADH beitragen. Zink trägt zudem zu normaler Fertilität und Reproduktion sowie einem funktionierenden Immunsystem bei. Es spielt auch eine Rolle bei der Zellteilung.

N-Acetyl-L-Cystein ist ein Vorläufer für die endogene Synthese von Glutathion und erhöht den Glutathionspiegel langfristig. Glutathion gehört zu den wichtigsten als Antioxidans wirkenden Stoffen im Körper. N-Acetyl-L-Cystein verbessert auch Endometriosesymptome durch Verringerung von Endometriose assoziierten Zysten.

Eine Mangel- bzw. Fehlernährung verbundene Zelldysfunktion wird zudem durch Nahrungsergänzungen wie Omega-3-Fettsäuren, D-Vitamine und Eisen ausgeglichen. Zudem haben Omega-3-Fettsäuren ebenfalls eine antientzündliche Wirkung auf den Zellstoffwechsel.

So besteht ein Zusammenhang zwischen Endometriose und dem Konsum von Nahrungsfetten. Durch die erhöhte Einnahme von langkettigen Omega-3-Fettsäuren kann das Risiko für Endometriose reduziert werden, indem die Entzündungsaktivität reduziert wird, sowie die Cytokinfunktion geregelt wird. Die Aufnahme von mehrfach ungesättigten Fettsäuren wie Omega-3-Fettsäuren trägt somit zur Gesunderhaltung bei und vermindert Entzündungsreaktionen.

D-Vitamine sind für die Absorption von Kalzium und Phosphat, das Immunsystem sowie die Entwicklung des Nerven- und Muskelsystems wichtig. Endometriosepatientinnen zeigen oft Kalziumstoffwechselstörungen. Ein D-Vitamindefizit ist auch oft mit Infertilität und einer erhöhten Fehlgeburtenrate verbunden. Allerdings sind Daten zu D-Vitaminspiegel von Endometriosepatientinnen inkonsistent. Nichtsdestotrotz wird durch das erfindungsgemäße Kombinationspräparat die Verabreichung annähernd der empfohlenen Tagesdosis verabreicht, weil D-Vitamine für die Reproduktion eine wichtige Rolle spielen und zunehmend klar ist, dass die Versorgung der Bevölkerung häufig kritisch ist. Ferner reguliert Vitamin D3 die Survivin Genexpression nieder. Survivin ist ein Apoptose-hemmendes Protein. Eine Änderung der Apoptose ist mit dem Wachstum und Überleben von Endometriosezellen an ektopischen Stellen verbunden.

Eisen trägt zur normalen Funktion des Immunsystems bei. Eine Eisensupplementation kann zudem generell das Risiko von Infertilität reduzieren.

Eine antientzündliche Behandlung der durch Cytokine, TNF, etc. induzierten Entzündung findet neben der Gabe von Omega-3-Fettsäuren auch durch Lycopen statt. Lycopen zeigt stark antientzündliche Eigenschaften, weil es die Produktion von entzündungsassoziierten Cytokinen verhindert. Zudem hat es auch antioxidative Eigenschaften. Dies wirkt sich positiv auf die bei Endometriosepatientinnen vorliegende erhöhte Entzündungsaktivität aus.

Schließlich kann Lycopen auch die Östrogenaktivität vermindern. Da, wie oben geschildert, bei Endometriose oft der Östrogenspiegel erhöht ist, ist Lycopen somit auch ein wichtiger Faktor um die hormonelle Situation von Endometriosepatientinnen zu verbessern. Es ist daher besonders vorteilhaft Lycopen in einer Menge von mindestens 8 mg im Kombinationspräparat zu verwenden, um die gewünschten Effekte auch tatsächlich zu erreichen. Eine zu geringe Menge von Lycopen zeigt nämlich nicht die antioxidativen, antientzündlichen und östrogenspiegelregulierenden Eigenschaften.

Ubiquinon-10, auch Coenzym-Q10 genannt, gleicht den Mangel an mitochondrialer Energie in den Eizellen aus, indem es die mitochondriale Aktivität der Eizelle unterstützt, welche mit zunehmendem Alter abnimmt. Besonders viele Mitochondrien befinden sich in Zellen mit hohem Energieverbrauch, wie eben auch den Eizellen. Die Mitochondrien bilden das energiereiche Molekül Adenosintriphosphat. Die weiblichen Keimzellen haben von allen Körperzellen die meisten Mitochondrien. Schädigungen der Mitochondrien führen einerseits zu einer geringeren Energiebereitstellung und andererseits zur Erhöhung von oxidativem Stress und somit zur Bildung von Sauerstoffradikalen in den Eizellen. Supplementiertes Coenzym-Q10 findet sich vor allem in der Nebenniere und den Eierstöcken. Die Coenzym-Q10 Konzentration reduziert sich mit zunehmendem Alter.

Magnesium ist wichtig für den Energiestoffwechsel und ist in viele Enzymreaktionen involviert. Während der Schwangerschaft liegt ein erhöhter Bedarf an Magnesium vor, weil mehr Magnesium ausgeschieden wird. Nichtsdestotrotz ist die Dosierung sehr wichtig, weil selbst bei sehr hohen Magnesiumgaben nur ein Bruchteil resorbiert wird.

Während einer Schwangerschaft ist der Bedarf an Jod erhöht. Jod trägt unter anderem auch zu einem normalen Energiestoffwechsel bei.

Durch das erfindungsgemäße Kombinationspräparat wird oxidativem Stress entgegen gewirkt, Entzündungsreaktionen gemildert, hormonelle Balance gefördert, ein Stoffwechselgleichgewicht und Zellhomöostase hergestellt, die Durchblutung und Zellentgiftung gefördert und die mitochondriale Energieerzeugung in den Eizellen unterstützt

Von Vorteil erweist sich dabei, dass N-Acetyl-L-Cystein in einem Verhältnis von 1:1 mit C-Vitamin im Kombinationspräparat enthalten ist, weil sonst eine überschießende Cystinbildung und damit eine verminderte Glutathionbildung stattfinden. Dies begründet sich dadurch, dass das C-Vitamin einer zu schnellen Oxidation des N-Acetyl-Cysteins zu Cystin entgegen wirken soll. Dies hat wiederum zwei Gründe: erstens trägt überschüssiges Cystin zu einer verstärkten Bildung von Nierensteinen bei entsprechend veranlagten Personen bei und zweitens fand man in einer in-vitro Studie aus dem Jahre 2009, dass Cystin wesentlich schlechter zur Bildung von Glutathion beiträgt als N-Acetylcystein. Auch bereits im Plasma vorhandenes Cystin wird erst durch die Reduzierung zu Cystein erst wieder effektiv dem Stoffwechselweg zur Glutathionsynthese zugeführt. Daher ist die Gabe von C-Vitamin in Kombination mit einem weiteren starken wasserlöslichen Antioxidans sehr wichtig für die Effektivität des erfindungsgemäßen Kombinationspräparats. Dies wird unterstützt durch eine weitere Studie von 2000, nach der die kombinierte Gabe von NAC und Vitamin C sogar protektive Effekte gegenüber kanzerogenen Substanzen zeigte.

Die Omega-3-Fettsäuren Eicosapentaensäure (EPA) zu Docosahexaensäure (DHA) sind in einem Verhältnis von 3 zu 2 enthalten. Omega-3-Fettsäuren beeinflussen das Immunsystem, indem das körpereigene Abwehrsystem eine befruchtete Eizelle leichter toleriert und somit wirken sich Omega-3-Fettsäuren vorteilhaft aus. Die Anzahl regulatorischer Immunzellen wird erhöht und dadurch akzeptiere der Körper Antigene (befruchtete Eizelle) leichter.

Als Omega-3-Fettsäuren werden vorzugsweise Eicosapentaensäure (EPA) und Docosahexaensäure (DHA) verwendet. Es kann aber auch α-Linolensäure (ALA) als Omega-3-Fettsäure im Kombinationspräparat verwendet werden. Vorzugsweise wird für die Herstellung des Kombinationspräparats Fischöl verwendet, wobei 18 % EPA und 12 % DHA sind. Es können Omega-3-Fettsäuren sowohl tierischer als auch pflanzlicher Herkunft verwendet werden.

Von Vorteil erweist sich zudem die quantitative Zusammensetzung des Kombinationspräparats wie C-Vitamin in Mengen von 25 mg bis 400 mg, insbesondere 50 mg bis 200 mg, vorzugsweise 100 mg, E-Vitamine in Mengen von 5 mg bis 80 mg, insbesondere 10 mg bis 40 mg, vorzugsweise 20 mg, B1-Vitamin in Mengen von 750 µg bis 12 mg, insbesondere 1,5 mg bis 6 mg, vorzugsweise 3 mg, B2-Vitamin in Mengen von 750 µg bis 12 mg, insbesondere 1,5 mg bis 6 mg, vorzugsweise 3 mg, B5-Vitamin in Mengen von 3 mg bis 50 mg, insbesondere 6 mg bis 25 mg, vorzugsweise 12 mg, B6-Vitamin in Mengen von 1 mg bis 20 mg, insbesondere 2 mg bis 10 mg, vorzugsweise 4 mg, B7-Vitamine in Mengen von 50 µg bis 1 mg, insbesondere 100 µg bis 500 µg, vorzugsweise 200 µg, B9-Vitamin in Mengen von 100 µg bis 5 mg, insbesondere 200 µg bis 2 mg, vorzugsweise 800 µg, B12-Vitamine in Mengen von 1 µg bis 50 µg insbesondere 2 µg bis 25 µg, vorzugsweise 7 µg, gegebenenfalls D-Vitamine in Mengen von 1 µg bis 50 µg, insbesondere 5 µg bis 20 µg, vorzugsweise 10 µg, Niacin in Mengen von 5 mg bis 200 mg, insbesondere 10 mg bis 100 mg, vorzugsweise 35 mg, Zink und/oder einer Zinkverbindung in Mengen von 1 mg bis 20 mg, insbesondere 2 mg bis 10 mg, vorzugsweise 5 mg, Eisen und/oder eine Eisenverbindung in Mengen von 1 mg bis 30 mg, insbesondere 2 mg bis 15 mg, vorzugsweise 7,5 mg, Selen und/oder eine Selenverbindung in Mengen von 20 µg bis 500 µg, insbesondere 50 µg bis 250 µg, vorzugsweise 100 µg, Kupfer und/oder eine Kupferverbindung in Mengen von 100 µg bis 5 mg, insbesondere 200 µg bis 3 mg, vorzugsweise 1 mg, Jod und/oder eine Jodverbindung in Mengen von 20 µg bis 800 µg, insbesondere 50 µg bis 400 µg, vorzugsweise 150 µg, N-Acetylcystein in Mengen von 25 mg bis 400 mg, insbesondere 50 mg bis 200 mg, vorzugsweise 100 mg, Lycopen in Mengen von 8 mg bis 100 mg, insbesondere 9 mg bis 50 mg, vorzugsweise 10 mg, Omega-3-Fettsäuren, insbesondere Eicosapentaensäure und Docosahexaensäure, jeweils in Mengen von 10 mg bis 500 mg, insbesondere 20 mg bis 250 mg, vorzugsweise 50 mg bis 100 mg, Ubiquinon-10 in Mengen von 5 mg bis 200 mg, insbesondere 10 mg bis 100 mg, vorzugsweise 35 mg, die mit dem Kombinationspräparat je verabreichter Tagesdosis eingenommen wird. Die Wirksamkeit des Kombinationspräparats beruht nicht nur auf der Wirkung ausgewählter Einzelsubstanzen, sondern der synergistischen Wirkung, insbesondere der antioxidativen Wirkung durch die zeitverzögerte Freisetzung von C-Vitamin, der antientzündlichen sowie östrogenspiegelregulierenden Wirkung von Lycopen sowie der synergistischen Wirkung von C-Vitaminen und N-Acetyl-L-Cystein, um eine überschießende Cystinbildung und damit verminderte Glutathionbildung zu verhindern. Die Entwicklung und Reifung der Eizelle wird durch die Anwesenheit der Menge von Sauerstoffradikalen beeinflusst. Durch die ausgewählten Mengen der oben genannten Wirkstoffe kann unter anderem der Wirkung der Sauerstoffradikale entgegen gewirkt werden und die Entwicklung positiv beeinflusst werden. Durch die Ausgewogenheit der ausgewählten Mengen des Kombinationspräparats kann der gewünschte Effekt erzielt werden.

Vorzugsweise ist Eicosapentaensäure (EPA) in Mengen von 10 mg bis 500 mg, insbesondere 10 mg bis 100 mg, vorzugsweise 90 mg und Docosahexaensäure (DHA) in Mengen von 10 mg bis 200 mg, insbesondere 10 mg bis 100 mg, vorzugsweise 60 mg, je verabreichter Tagesdosis im Kombinationspräparat enthalten, wodurch ein positiver Einfluss auf viele Stoffwechselfunktionen erfolgt. Es wird unter anderem zur Gesundheit, insbesondere zu normalen Augen- und Gehirnentwicklung des Ungeborenen beigetragen.

Ferner kann sich als vorteilhaft erweisen, wenn noch weitere Wirkstoffe wie Magnesium, Vitamin K, Molybdän, Nikotinsäureamid, Chrom, Kalzium, β-Carotin, Probiotika, Phosphor, Mangan und/oder Vitamin A enthalten sind. Insbesondere durch die Zugabe von Magnesium zum Kombinationspräparat können die Erfolgsaussichten für eine Schwangerschaft nach einer In-vitro-Fertilisation durch die Verabreichung des erfindungsgemäßen Kombinationspräparats weiter erhöht werden.

Besonders vorteilhaft dabei erweist sich, wenn Magnesium und/oder eine Magnesiumverbindung in Mengen von 5 mg bis 500 mg, insbesondere 10 mg bis 250 mg, vorzugsweise 30 mg bis 100 mg, Vitamin K in Mengen von 0,001 mg bis 0,5 mg, insbesondere 0,01 mg bis 0,1 mg, vorzugsweise 0,05 mg bis 0,08 mg, Molybdän in Mengen von 0,001 mg bis 0,5 mg, insbesondere 0,01 mg bis 0,1 mg, vorzugsweise 0,05 mg bis 0,08 mg, Nikotinsäureamid in Mengen von 8 mg bis 100 mg, insbesondere 5 mg bis 40 mg, vorzugsweise 10 mg, Chrom in Mengen von 0,001 mg bis 0,5 mg, insbesondere 0,01 mg bis 0,1 mg, vorzugsweise 0,05 mg bis 0,08 mg, Kalzium in Mengen von 25 mg bis 400 mg, insbesondere 50 mg bis 200 mg, vorzugsweise 100 mg, β-Carotin in Mengen von 750 µg bis 12 mg, insbesondere 1,5 mg bis 6 mg, vorzugsweise 3 mg, Probiotika in Mengen von 100 µg bis 10 mg, insbesondere 200 µg bis 3 mg, vorzugsweise 1 mg, Phosphor in Mengen von 5 mg bis 500 mg, insbesondere 10 mg bis 250 mg, vorzugsweise 30 mg bis 100 mg, Mangan in Mengen von 100 µg bis 5 mg, insbesondere 200 µg bis 3 mg, vorzugsweise 1 mg, Vitamin A in Mengen von 20 µg bis 500 µg, insbesondere 50 µg bis 250 µg, vorzugsweise 100 µg, im Kombinationspräparat enthalten sind.

Zum besseren Verständnis der Erfindung wird diese anhand der nachfolgenden Ausführungsbeispiele näher erläutert.

Im Sinne der Erfindung werden unter pre-A-, B-, C-, D- und E-Vitaminen alle zu diesen Vitaminen bekannten Vertreter verstanden. So sollen beispielsweise durch E-Vitamine Tocopherole, Tocotrienole und Tocomonoenole umfasst werden. Durch die Bezeichnung D-Vitamine sind insbesondere Vitamin D3 (Cholecalilferol), 25(OH) Vitamin D3, Vitamin D2 (Ergocalciferol) und Calcitriol umfasst. Dies sei nur beispielhaft erwähnt und gilt auch für die anderen in der Erfindung genannten Vitamine.

Die Formulierung "Lycopen in einer Menge mit einer Untergrenze von 8 mg je mit dem Kombinationspräparat verabreichter Tagesdosis enthalten", ist im Sinne der Erfindung so zu verstehen, dass bei Verabreichung einer Kapsel die Menge in der einen Kapsel enthalten ist und bei Verabreichung von 2 oder mehr Kapseln die Menge auf die Anzahl der Kapseln gleich aufgeteilt ist.

Bei den Mineralstoffen und Spurenelementen des Kombinationspräparats sind im Sinne der Erfindung auch deren Derivate bzw. Salze zu verstehen, auch wenn nicht immer explizit angeführt.

Als Darreichungsform werden vorzugsweise feste Arzneiformen, wie Tabletten oder Kapseln, gewählt. Alternativ können aber auch flüssige oder halbfeste Arzneiformen, wie Suppositorien, gewählt werden.

Das erfindungsgemäße Kombinationspräparat ist speziell für die Bedürfnisse des weiblichen Körpers von Endometriosepatientinnen bei Kinderwunsch und Schwangerschaft entwickelt. Es kann sowohl ein Nahrungsergänzungsmittel, ein diätetisches Lebensmittel für besondere medizinische Zwecke (ergänzende bilanzierte Diät), als auch ein Arzneimittel sein. Dadurch ist für den Anwender gewähr-leistet, dass bei der Zulassung als Arzneimittel bzw. bilanzierte Diät das Präparat zumindest eine klinische Studie durchlaufen hat und bei der Zulassung als Nahrungsergänzungsmittel ein einfacher und gegebenenfalls diskreter Zugang zum Präparat gegeben ist.

Das Kombinationspräparat wird vorzugsweise oral eingenommen. Das Präparat kann sowohl als Human- als auch Veterinärprodukt verwendet werden.

Das Kombinationspräparat umfasst zumindest die Wirkstoffe, wobei das Kombinationspräparat aus einer oder mehreren Darreichungsformen bestehen kann:
Vitamine C, B1, B2, B3, B5, B7, B9, B12, und E
gegebenenfalls Vitamin D3
Ubiqinon-10
Lycopen bzw. Lycopin
Acetyl L- Cystein
Zink, Eisen, Kupfer, Selen, Jod, Magnesium
Omega-3-Fettsäuren Eicosapentaensäure (EPA) und Docosahexaensäure (DHA)

Die im Kombinationspräparat enthaltenen Wirkstoffe liegen vorzugsweise in nachfolgend angeführter Form vor: Vitamin C als Ascorbinsäure gecoatet um eine zeitverzögerte Freisetzung von C-Vitamin zu bewirken, Vitamin B1 als Thiamin HCl, Vitamin B2 wird auch als Riboflavin bezeichnet, Vitamin B5 als Calcium-D-panthothenat, Vitamin B6 als Pyridoxin HCl, Vitamin B12 als Cyanocobalamin auf Mannit, Vitamin B9 wird auch als Folsäure bezeichnet, Vitamin B3/Niacin als Nicotinsäureamid, Vitamin B7 wird auch Biotin bezeichnet, Zink als Zinkglukonat, Eisen als Eisen(II)sulfat, Kupfer als Kupfersulfat, Selen als Natriumselenit in Calciumcarbonat, Ubichinon-10 wird auch als Coenzym Q10 bezeichnet, Acetyl L-Cystein als N-Acetyl-L-Cystein, Lycopen als Pulver umfassend Lycopin und modifizierte Stärke, Magnesium als Magnesiumoxid, Jod als Kaliumiodid, Vitamin E als alpha-Tocopherylacetat bzw. vorzugsweise als D-Alpha-Tocopherylsuccinat, Vitamin D3 als Cholecalciferol, vor. Es können aber auch andere als die vorab beschriebenen Formen, insbesondere Salze, der Wirkstoffe verwendet werden. Diese Wirkstoffe bzw. Zutaten liegen vorzugsweise in einer Kapsel mit Pulvermischung vor.

Eicosapentaensäure und Docosahexaensäure liegen vorzugsweise in einer Fischölkapsel vor.

Es können aber noch weitere Wirkstoffe, insbesondere Mineralstoffe und Spurenelemente, Probiotika und weitere Vitamine, etc. im Kombinationspräparat enthalten sein.

In der nachfolgenden Tabelle sind die Mengenbereiche der mit dem Kombinationspräparat verabreichten empfohlenen Tagesdosis für die Steigerung der Fertilität bei Endometriosepatientinnen der einzelnen für die Erfindung relevanten Wirkstoffe angeführt. Jedenfalls enthält das Kombinationspräparat die Wirkstoffe C-Vitamin, E-Vitamine, B1-Vitamin, B2-Vitamin, B5-Vitamin, B6-Vitamine, B7-Vitamin, B9-Vitamin, B12-Vitamine, D-Vitamine, Niacin, Zink, Eisen, Selen, Jod, Kupfer, N-Acetylcystein, Lycopen, Omega-3-Fettsäuren und Ubiquinon-10. Bei den weiteren angeführten Wirkstoffen, die optional enthalten sein können, sind ebenfalls jeweils Unter- und Obergrenzen angeführt. Vorzugsweise wird das erfindungsgemäße Kombinationspräparat als eine Kapsel pro Tag eingenommen. Es können allerdings die Wirkstoffmengen so gewählt werden, dass zwei Kapseln pro Tag erforderlich sind, um die empfohlene Dosierung zu erreichen. Es ist auch möglich, dass einzelne Wirkstoffe, wie z.B. die Omega-3-Fettsäuren, in einer anderen Kapsel wie die übrigen Wirkstoffe verpackt sind und somit das Kombinationspräparat von mehreren Darreichungsformen, vorzugsweise zwei Kapseln, gebildet ist.

| **Wirkstoff** | **empfohlener Mengenbereich in mg pro Tag** |
|---|---|
| Vitamin C | 25 -400 |
| Vitamin E | 5 - 80 |
| Vitamin B1 | 0,75 - 12 |
| Vitamin B2 | 0,75 - 12 |
| Vitamin B5 | 3 - 50 |
| Vitamin B6 | 1 - 20 |
| Vitamin B12 | 0,001 - 0,05 |
| Vitamin B9 | 0,1 - 5 |
| Vitamin D | 0 - 0,05 |
| Vitamin B3 | 5 - 200 |
| Vitamin B7 | 0,05 - 1 |
| Zink | 1 - 20 |
| Jod | 0,02 - 0,8 |
| Eisen | 1 - 30 |
| Kupfer | 0,1 - 5 |
| Selen | 0,02 - 0,5 |
| Ubiquinon-10 | 5 - 200 |
| N-Acetyl-L-Cystein | 25 - 400 |
| Lycopen | 8 - 100 |
| EPA | 10 - 500 |
| DHA | 10 - 500 |
| Magnesium | 0 - 500 |
| Vitamin K | 0 - 0,1 |
| Molybdän | 0 - 0,1 |
| Chrom | 0 - 0,1 |
| Calcium | 0 - 400 |
| β-Carotin | 0 - 5 |
| Probiotika | 0 - 10 |
| Phosphor | 0 - 150 |
| Mangan | 0 - 3 |
| Vitamin A | 0 - 0,5 |

Sehr gute Ergebnisse bezüglich der Fertilitätssteigerung bei Endometriosepatientinnen zeigt die Wirkstoffkombination C-Vitamin, E-Vitamine, B1-Vitamin, B2-Vitamin, B5-Vitamin, B6-Vitamine, B7-Vitamin, B9-Vitamin, B12-Vitamine, D-Vitamine, Niacin, Zink, Eisen, Selen, Jod, Kupfer, N-Acetylcystein, Lycopen, Omega-3-Fettsäuren, gegebenenfalls Magnesium und Ubiquinon-10, wobei Lycopen in einer Menge mit einer Untergrenze von 8 mg im Kombinationspräparat enthalten ist. Die Wirkstoffe, bis auf die Omega-3-Fettsäuren, liegen vorzugsweise pulverförmig vor und sind in einer Darreichungsform, vorzugsweise einer Kapsel, enthalten. Es zeigt sich von Vorteil, wenn EPA und DHA in einer eigenen Darreichungsform, vorzugsweise ebenfalls in einer Kapsel, verpackt sind aber in Kombination mit der erst genannten Darreichungsform verabreicht werden.

Im nachfolgenden sind Ausführungsbeispiele zum Kombinationspräparat der therapeutisch wirksamen Wirkstoffe des erfindungsgemäßen Kombinationspräparats angeführt, welche die vorteilhaften Wirkungen erzielen.

Es sind auch Exzipienten, wie Hilfsstoffe, Füllstoffe, Trägerstoffe, Bindemittel, etc., welche zur technischen Verarbeitbarkeit des erfindungsgemäßen Kombinationspräparats erforderlich sind, enthalten. Diese Exzipienten haben jedoch keine therapeutische Wirksamkeit und keine positiven Effekte auf die gesteigerte Fertilität von Endometriosepatientinnen und sind dementsprechend auch keine therapeutisch wirksamen Wirkstoffe im Sinne der Erfindung.

Die nachfolgende Tabelle zeigt Ausführungsbeispiele unter Angabe der Menge des Kombinationspräparats in absoluten Zahlen der therapeutisch wirksamen Wirkstoffe. Die Mengenangaben beziehen sich auf die Menge der Wirkstoffe und nicht auf die Menge der für die Herstellung des Kombinationspräparats verwendeten Formen des Wirkstoffes.

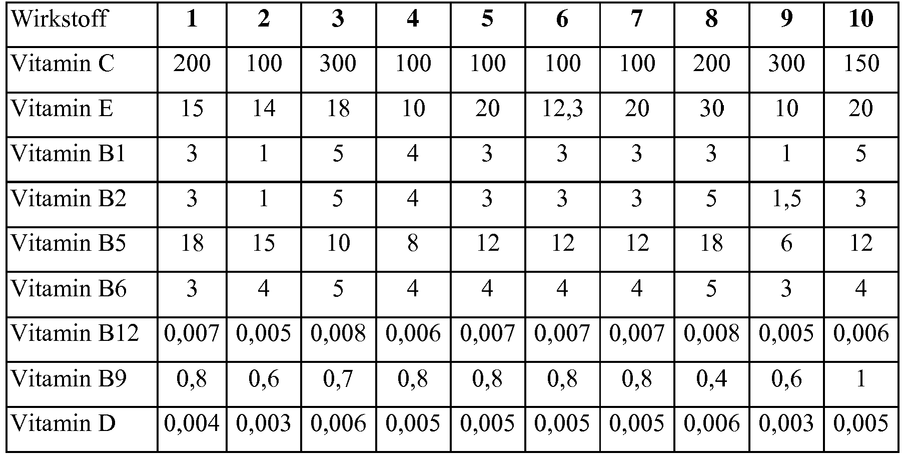

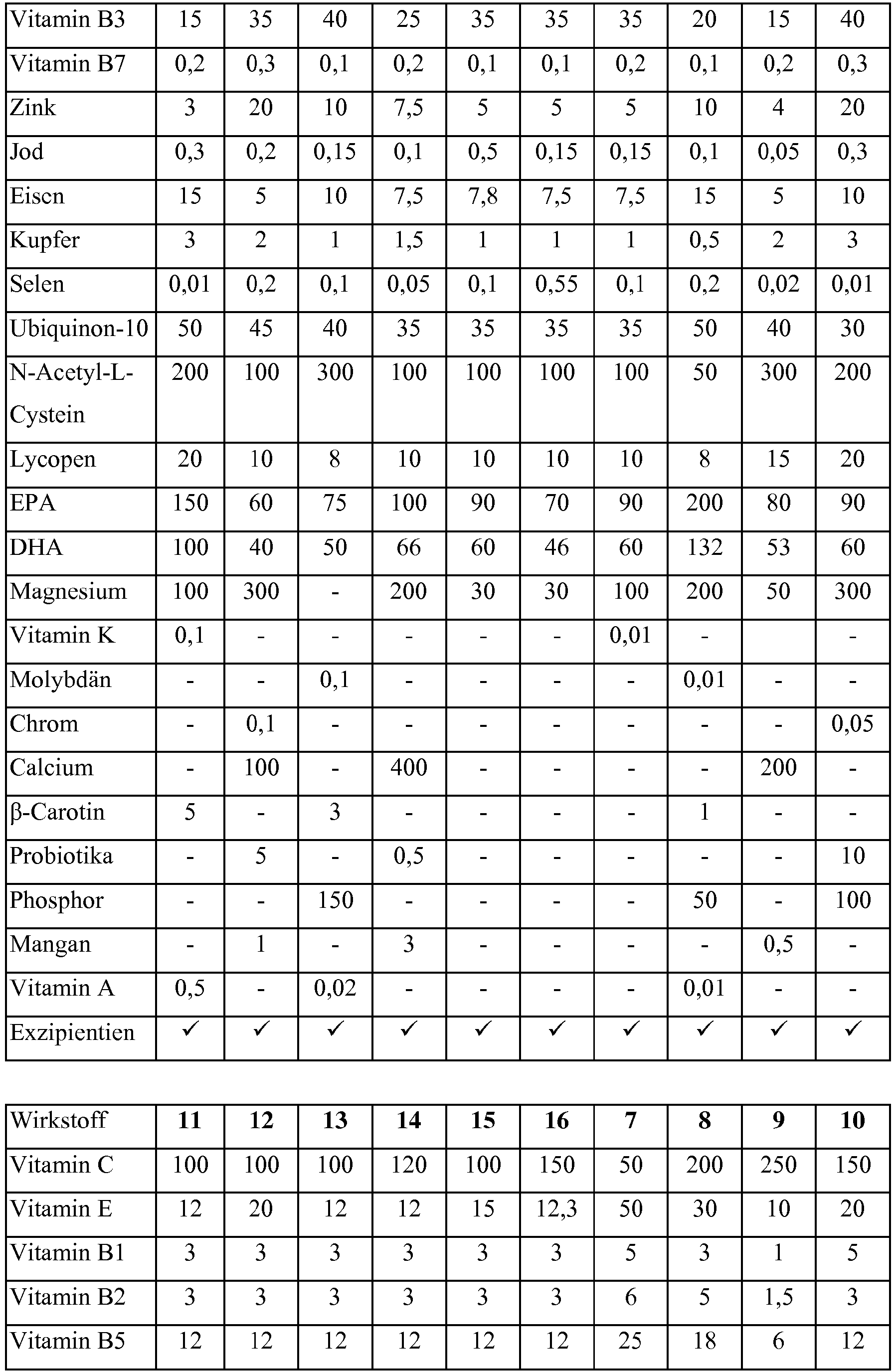

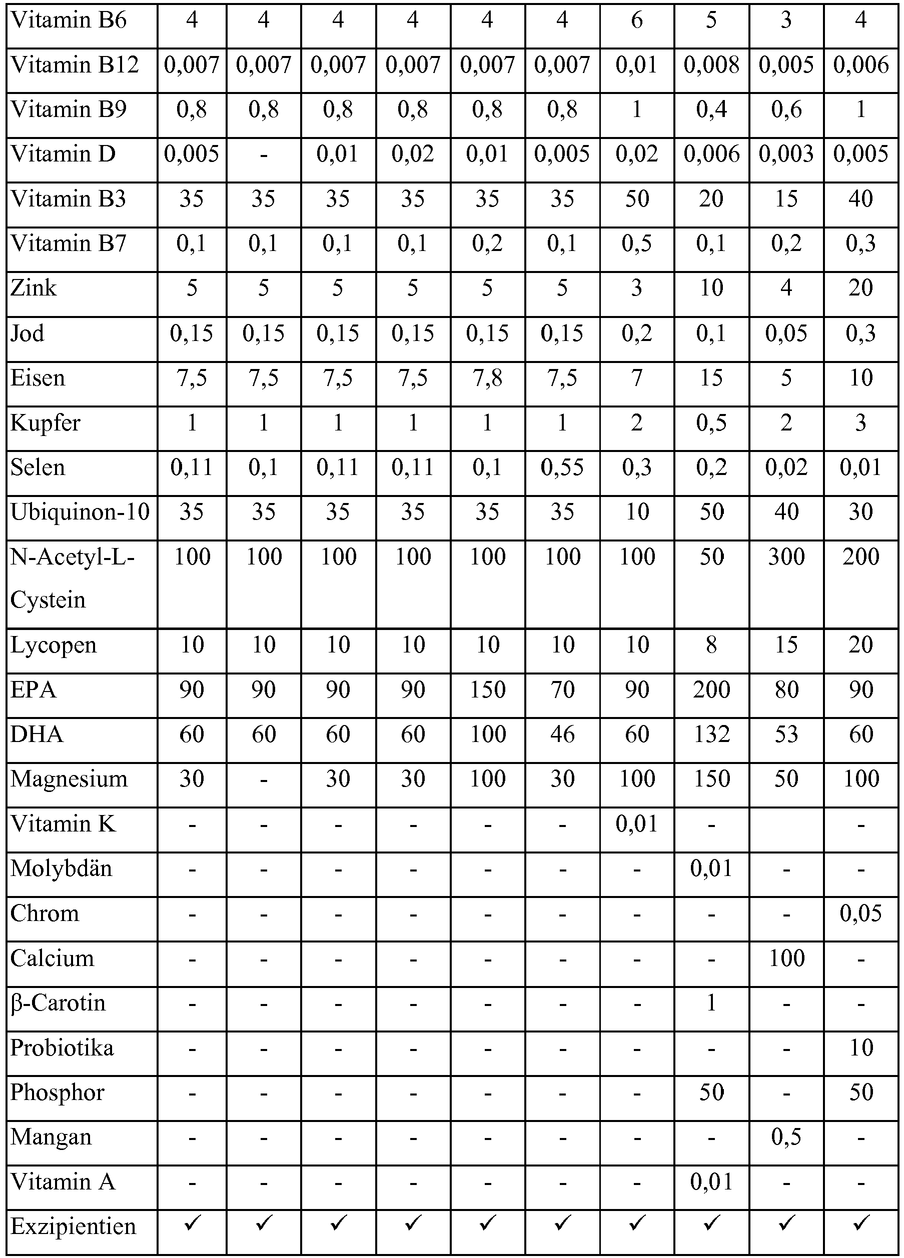

In einer Pilotstudie konnte die Wirksamkeit des erfindungsgemäßen Kombinationspräparats im Vergleich zum aus dem Stand der Technik bekannten Kombinationspräparat der Anmelderin ohne N-Acetyl-L-Cystein sowie ohne Lycopen bei Endometriosepatientinnen nachgewiesen werden. Es konnte gezeigt werden, dass in der Gruppe der Endometriosepatientinnen, die das aus dem Stand der Technik bekannte Präparat erhielten, 30 % der Frauen, und in der Gruppe, welche das erfindungsgemäße Kombinationspräparat 5, 6 und 7 erhielten, 55% der Frauen nach reproduktionsmedizinischer Behandlung einen positiven Schwangerschaftstest zeigten. Zudem konnte auch ein signifikanter Unterschied in der Rate der Blastozystenbildung gezeigt werden.
Ferner zeigte eine Beobachtungsstudie mit n=20 Patientinnen mit Endometriose in reproduktionsmedizinischer Behandlung (IVF/ICSI), die erfolgreiche Behandlung mit einem Kombinationspräparat gemäß Beispiel 11, 13 und 14. Verglichen wurden bei den gleichen Patientinnen deren Vorzyklen, bei denen mit einem bereits bekannten Präparat, nämlich Fertilovit® F, F 35plus oder FThy der Anmelderin, je nach Alter und Schilddrüsensituation der Patientin, supplementiert wurde mit einem Zyklus, bei dem zusätzlich unter anderem noch N-Acetyl-L-Cystein (NAC) und Lycopen supplementiert wurde.

Nachfolgend ist das Ergebnis dieser Beobachtungsstudie tabellarisch dargestellt:

| | Vorzyklen | Zyklus mit zusätzlicher NAC Supplementierung | Signifikanz |
|---|---|---|---|
| Alter | 36,7 +/- 4,0 | | |
| Vorversuche | 3,4 | | |
| | | | |
| Stimulationsprotokoll | 19xLP; 1xSP | 19xLP; 1xSP | |
| Stimulationsdosis | 2797,5 +/-974,3 IU | 3000 +/- 1298,3 IU | p:0,59 |
| Oocyten | 11,4 +/- 5,6 | 9,6 +/-5,4 | p: 0,45 |
| MII Oocyten | 9,3 +/- 5,2 | 8,1 +/- 4,3 | p: 0,84 |
| 2PN | 6,4 +/- 3,1 | 6,1 +/- 3,3 | p: 0,84 |
| Befruchtungsrate | 68,8 % | 74,7 % | p: 0,23 |
| Blastocysten | 2,6 +/- 2,3 | 3,2 +/- 2,4 | p: 0,74 |
| Blastocystenrate | 39,8 % | 52,9 % | p: 0,04 |
| Top-Blastocysten | 0,35 +/- 0,72 | 1,2 +/- 1,6 | |
| Chemische Schwangerschaft | 6 | 11 | |
| Schwangerschaftsrate | 0,3 | 0,55 | |
| Positive Herzaktion | 2 | 11 | |
| Rate | 0,1 | 0,55 | |
| Geburt | 1 | 9 | |
| Geburtenrate | 0,05 | 0,45 | |

Wie das Ergebnis der Beobachtungsstudie zeigt, ist bei den Schwangerschaftsraten ein deutlicher Anstieg und bei der Blastozystenrate ein signifikanter Anstieg nach Einnahme des erfindungsgemäßen Kombinationspräparats zu beobachten.

Von Vorteil dabei zeigt sich, dass durch die Kombination der oben genannten Wirkstoffe im Kombinationspräparat deren synergistische Wirkung zur Geltung kommt. Durch die genannte Kombination der Wirkstoffe wird dem weiblichen Organismus von Endometriosepatientinnen ein Optimum an Mikronutrienten zur Verfügung gestellt, um die Eizellqualität zu verbessern, die Empfängnisbereitschaft zu erhöhen und das Risiko einer Fehlgeburt zu reduzieren.

Obwohl die Ätiologie von Endometriose noch kaum verstanden ist, sind doch bereits ein paar Risikofaktoren definiert. Ein erhöhter Vitamin-D Plasmaspiegel und eine erhöhter Konsum von Milchprodukten sind mit einem geringeren Risiko Endometriose zu entwickeln verbunden. Es wird postuliert, dass Vitamin D dadurch wirkt, die Survivin-Genexpression hinunter zu regulieren und somit das Überleben von Endometriosegewebe vermindert wird. Survivin wird in erhöhter Expression in ektopischen endometrischen Stromazellen gefunden und kann ein Entkommen von Apoptose der Endometriosezellen begünstigen. Die Genexpression von Survivin in ektopische Endometriosegewebe ist signifikant höher als jene im eutopischen Endometrium von Patientinnen mit und ohne Endometriose während eines Zyklus. Somit sind Inhibitoren von Survivin bzw. der Survivinexpression bei Endometriosepatientinnen zu bevorzugen. In dem Kombinationspräparat nach der Erfindung, ergibt sich ein synergistischer Effekt, durch die Kombination von Vitamin D mit Vitamin E und Selen sowohl auf ernährungsphysiologischer als auch pharmakologischer Ebene. Vitamin E und Selen gelten als Inhibitoren des Survivin. Vitamin D reguliert die Survivin-Expression hinunter. Dieser synergistische Effekt des Kombinationspräparats trägt zur Reduktion des Wachstums des ektopischen Endometriosegewebes bei ohne dabei das eutopische Gewebe, welches für eine erfolgreiche Implantation erforderlich ist, negativ zu beeinflussen.

In einer bevorzugten Ausführungsvariante besteht das erfindungsgemäße Kombinationspräparat aus pflanzlicher Stärke, Überzugsmittel Hydroxypropylmethylcellulose (Kapselhülle), L-Ascorbinsäure, N-Acetyl-L-Cystein, Magnesiumoxid, Zinkglukonat, Coenzym Q10, Nikotinsäureamid, Calciumcarbonat, Eisen (II) sulfat, DL-alpha-Tocopherylacetat bzw. D-alpha Tocopherylsuccinat, Siliciumdioxid, Calcium-D-Pantothenat, Trennmittel Magnesiumstearat bzw. Magnesiumsalze von Speisefettsäuren, Pyridoxin HCl, Thiamin HCl, Kupfersulfat, Riboflavin, Farbstoff Titandioxid (Kapselhülle), Pteroylmonoglutaminsäure, Kaliumjodid, Natriumselenit, D-Biotin, Cyanocobalamin, Cholechalciferol, Lycopen und Folsäure. Sind die Omega-3-Fettsäuren eigens verpackt bzw. einzunehmen umfasst die Omega-3-Kapsel Fischöl mit den Omega-3-Fettsäuren EPA und DHA in einem Verhältnis von 3:2, Gelatine, Glycerin, Wasser und als Stabilisator D-alpha Tocopherol.

Die positive Wirkung des erfindungsgemäßen Kombinationspräparats kann sowohl in Vorbereitung für eine natürliche Schwangerschaft als auch in Vorbereitung für eine Insemination, in-vitro Fertilisation als auch intracytoplasmatische Spermieninjektion genutzt werden.

Vorzugsweise sind die Ausführungsvarianten des Kombinationspräparats frei von Gluten und Laktose.

Alle Ausführungsvarianten des Kombinationspräparats sollten einmal täglich in Form einer Kapsel bzw. einer Kapsel in Kombination mit einer Omega-3-Kapsel oral eingenommen werden. Die empfohlene Mindesteinnahmedauer beträgt 1 bis 3 Monate. Das Präparat sollte dann bei Eintreten einer Schwangerschaft zumindest bis in die 12. Schwangerschaftswoche weiterhin eingenommen werden.

Die Ausführungsbeispiele zeigen mögliche Ausführungsvarianten des Kombinationspräparats, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Ferner können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

## Patentansprüche

1. Kombinationspräparat zur Anwendung in einem Verfahren für die Behandlung Endometriose assoziierter weiblicher Sub- und Infertilität umfassend zumindest die Wirkstoffe C-Vitamin, E-Vitamine, B1-Vitamin, B2-Vitamin, B5-Vitamin, B6-Vitamine, B7-Vitamin, B9-Vitamin, B12-Vitamine, Niacin, Zink, Eisen, Selen, Jod, Kupfer, N-Acetylcystein, Lycopen, Omega-3-Fettsäuren, Ubiquinon-10, und gegebenenfalls D-Vitamine, **dadurch gekennzeichnet, dass** Lycopen in einer Menge mit einer Untergrenze von 8 mg im Kombinationspräparat enthalten ist.

2. Kombinationspräparat zur Anwendung gemäß Anspruch 1, **dadurch gekennzeichnet, dass** C-Vitamin und N-Acetylcystein in einem Verhältnis von 1:1 enthalten ist.

3. Kombinationspräparat zur Anwendung gemäß Anspruch 1 oder 2, **dadurch gekennzeichnet, dass** die Omega-3-Fettsäuren Eicosapentaensäure (EPA) zu Docosahexaensäure (DHA) in einem Verhältnis von 3:2 enthalten sind.

4. Kombinationspräparat zur Anwendung gemäß zumindest einem der Ansprüche 1 bis 3, **dadurch gekennzeichnet, dass** C-Vitamin in Mengen von 25 mg bis 400 mg, insbesondere 50 mg bis 200 mg, vorzugsweise 100 mg, E-Vitamine in Mengen von 5 mg bis 80 mg, insbesondere 10 mg bis 40 mg, vorzugsweise 12 mg, B1-Vitamin in Mengen von 750 µg bis 12 mg, insbesondere 1,5 mg bis 6 mg, vorzugsweise 3 mg, B2-Vitamin in Mengen von 750 µg bis 12 mg, insbesondere 1,5 mg bis 6 mg, vorzugsweise 3 mg, B5-Vitamin in Mengen von 3 mg bis 50 mg, insbesondere 6 mg bis 25 mg, vorzugsweise 12 mg, B6-Vitamin in Mengen von 1 mg bis 20 mg, insbesondere 2 mg bis 10 mg, vorzugsweise 4 mg, B7-Vitamine in Mengen von 50 µg bis 1 mg, insbesondere 100 µg bis 500 µg, vorzugsweise 100 µg, B9-Vitamin in Mengen von 100 µg bis 5 mg, insbesondere 200 µg bis 2 mg, vorzugsweise 800 µg, B12-Vitamine in Mengen von 1 µg bis 50 µg insbesondere 2 µg bis 25 µg, vorzugsweise 7 µg, gegebenenfalls D-Vitamine in Mengen von 1 µg bis 50 µg, insbesondere 5 µg bis 20 µg, vorzugsweise 10 µg, Niacin in Mengen von 5 mg bis 200 mg, insbesondere 10 mg bis 100 mg, vorzugsweise 35 mg, Zink und/oder eine Zinkverbindung in Mengen von 1 mg bis 20 mg, insbesondere 2 mg bis 10 mg, vorzugsweise 5 mg, Eisen und/oder eine Eisenverbindung in Mengen von 1 mg bis 30 mg, insbesondere 2 mg bis 15 mg, vorzugsweise 7,5 mg, Selen und/oder eine Selenverbindung in Mengen von 20 µg bis 500 µg, insbesondere 50 µg bis 250 µg, vorzugsweise 100 µg, Kupfer und/oder eine Kupferverbindung in Mengen von 100 µg bis 5 mg, insbesondere 200 µg bis 3 mg, vorzugsweise 1 mg, Jod und/oder eine Jodverbindung in Mengen von 20 µg bis 800 µg, insbesondere 50 µg bis 400 µg, vorzugsweise 150 µg, N-Acetylcystein in Mengen von 25 mg bis 400 mg, insbesondere 50 mg bis 200 mg, vorzugsweise 100 mg, Lycopen in Mengen von 8 mg bis 100 mg, insbesondere 5 mg bis 40 mg, vorzugsweise 10 mg, Omega-3-Fettsäuren, insbesondere Eicosapentaensäure und Docosahexaensäure, jeweils in Mengen von 10 mg bis 500 mg, insbesondere 30 mg bis 200 mg, vorzugsweise 50 bis 100 mg, Ubiquinon-10 in Mengen von 5 mg bis 200 mg, insbesondere 10 mg bis 100 mg, vorzugsweise 35 mg, im Kombinationspräparat enthalten sind.

5. Kombinationspräparat zur Anwendung gemäß zumindest einem der Ansprüche 1 bis 4, **dadurch gekennzeichnet, dass** Eicosapentaensäure in Mengen von 10 mg bis 500 mg, insbesondere 50 mg bis 300 mg, vorzugsweise 90 mg und Docosahexaensäure in Mengen von 10 mg bis 200 mg, insbesondere 40 mg bis 100 mg, vorzugsweise 60 mg, im Kombinationspräparat enthalten ist.

6. Kombinationspräparat zur Anwendung gemäß einem der Ansprüche 1 bis 5, **dadurch gekennzeichnet, dass** weitere Wirkstoffe wie Magnesium, Vitamin K, Molybdän, Nikotinsäureamid, Chrom, Kalzium, β-Carotin, Probiotika, Phosphor, Mangan und/oder Vitamin A enthalten sind.

7. Kombinationspräparat zur Anwendung gemäß Anspruch 6, **dadurch gekennzeichnet, dass** Magnesium und/oder eine Magnesiumverbindung in Mengen von 5 mg bis 500 mg, insbesondere 10 mg bis 250 mg, vorzugsweise 30 mg bis 100 mg, Vitamin K in Mengen von 0,001 mg bis 0,5 mg, insbesondere 0,01 mg bis 0,1 mg, vorzugsweise 0,05 mg bis 0,08 mg, Molybdän in Mengen von 0,001 mg bis 0,5 mg, insbesondere 0,01 mg bis 0,1 mg, vorzugsweise 0,05 mg bis 0,08 mg, Nikotinsäureamid in Mengen von 8 mg bis 100 mg, insbesondere 5 mg bis 40 mg, vorzugsweise 10 mg, Chrom in Mengen von 0,001 mg bis 0,5 mg, insbesondere 0,01 mg bis 0,1 mg, vorzugsweise 0,05 mg bis 0,08 mg, Kalzium in Mengen von 25 mg bis 400 mg, insbesondere 50 mg bis 200 mg, vorzugsweise 100 mg, β-Carotin in Mengen von 750 µg bis 12 mg, insbesondere 1,5 mg bis 6 mg, vorzugsweise 3 mg, Probiotika in Mengen von 100 µg bis 10 mg, insbesondere 200 µg bis 3 mg, vorzugsweise 1 mg, Phosphor in Mengen von 5 mg bis 500 mg, insbesondere 10 mg bis 250 mg, vorzugsweise 30 mg bis 100 mg, Mangan in Mengen von 100 µg bis 5 mg, insbesondere 200 µg bis 3 mg, vorzugsweise 1 mg, Vitamin A in Mengen von 20 µg bis 500 µg, insbesondere 50 µg bis 250 µg, vorzugsweise 100 µg, im Kombinationspräparat enthalten sind.

8. Kombinationspräparat zur Anwendung in einem Verfahren für die Behandlung Endometriose assoziierter weiblicher Sub- und Infertilität umfassend zumindest C-Vitamin, E-Vitamine, B1-Vitamin, B2-Vitamin, B5-Vitamin, B6-Vitamine, B7-Vitamin, B9-Vitamin, B12-Vitamine, Niacin, Zink, Eisen, Selen, Jod, Kupfer, Magnesium, N-Acetylcystein, Lycopen, Omega-3-Fettsäuren, Ubiquinon-10, D-Vitamine, **dadurch gekennzeichnet, dass** Lycopen in einer Menge mit einer Untergrenze von 8 mg im Kombinationspräparat enthalten ist.

9. Kombinationspräparat zur Anwendung gemäß Anspruch 8 zur Verwendung in der Behandlung Endometriose assoziierter weiblicher Sub- und Infertilität, **dadurch gekennzeichnet, dass** zumindest 100 mg C-Vitamin, 12 mg E-Vitamine, 3 mg B1-Vitamin, 3 mg B2-Vitamin, 12 mg B5-Vitamin, 4 mg B6-Vitamine, 100 µg B7-Vitamin, 800 µg B9-Vitamin, 7µg B12-Vitamine, 35 mg Niacin, 5 mg Zink, 7,7 mg Eisen, 110 µg Selen, 150 µg Jod, 1 mg Kupfer, 30 mg Magnesium, 100 mg N-Acetylcystein, 10 mg Lycopen, 35 mg Ubiquinon-10 und 20 µg D-Vitamine in einer Darreichungsform sowie 150 mg Omega-3-Fettsäuren in einer weiteren Darreichungsform als Wirkstoffe das Kombinationspräparat bilden.

## Claims

1. A combination preparation for use in a method for the treatment of female sub- and infertility associated with endometriosis, comprising at least the active ingredients C vitamin, E vitamins, B1 vitamin, B2 vitamin, B5 vitamin, B6 vitamins, B7 vitamin, B9 vitamin, B12 vitamins, niacin, zinc, iron, selenium, iodine, copper, N-acetylcysteine, lycopene, omega-3 fatty acids, ubiquinone-10, and optionally D vitamins, **characterized in that** lycopene is contained in the combination preparation in an amount with a lower limit of 8 mg.

2. The combination preparation for use according to claim 1, **characterized in that** C vitamin and N-acetylcysteine are contained in a ratio of 1:1.

3. The combination preparation for use according to claim 1 or 2, **characterized in that** the omega-3 fatty acids eicosapentaenoic acid (EPA) to docosahexaenoic acid (DHA) are contained in a ratio of 3:2.

4. The combination preparation for use according to at least one of claims 1 to 3, **characterized in that** C vitamin in amounts of 25 mg to 400 mg, in particular 50 mg to 200 mg, preferably 100 mg, E-vitamins in amounts of 5 mg to 80 mg, in particular 10 mg to 40 mg, preferably 12 mg, B1 vitamin in amounts of 750 µg to 12 mg, in particular 1.5 mg to 6 mg, preferably 3 mg, B2 vitamin in amounts of 750 µg to 12 mg, in particular 1.5 mg to 6 mg, preferably 3 mg, B5 vitamin in amounts of 3 mg to 50 mg, in particular 6 mg to 25 mg, preferably 12 mg, B6 vitamin in amounts of 1 mg to 20 mg, in particular 2 mg to 10 mg, preferably 4 mg, B7 vitamins in amounts of 50 µg to 1 mg, in particular 100 µg to 500 µg, preferably 100 µg, B9 vitamin in amounts of 100 µg to 5 mg, in particular 200 µg to 2 mg, preferably 800 µg, B12 vitamins in amounts of 1 µg to 50 µg, in particular 2 µg to 25 µg, preferably 7 µg, optionally D vitamins in amounts of 1 µg to 50 µg, in particular 5 µg to 20 µg, preferably 10 µg, niacin in amounts of 5 mg to 200 mg, in particular 10 mg to 100 mg, preferably 35 mg, zinc and/or a zinc compound in amounts of 1 mg to 20 mg, in particular 2 mg to 10 mg, preferably 5 mg, iron and/or an iron compound in amounts of 1 mg to 30 mg, in particular 2 mg to 15 mg, preferably 7.5 mg, selenium and/or a selenium compound in amounts of 20 µg to 500 µg, in particular 50 µg to 250 µg, preferably 100 µg, copper and/or a copper compound in amounts of 100 µg to 5 mg, in particular 200 µg to 3 mg, preferably 1 mg, iodine and/or an iodine compound in amounts of 20 µg to 800 µg, in particular 50 µg to 400 µg, preferably 150 µg, N-acetylcysteine in amounts of 25 mg to 400 mg, in particular 50 mg to 200 mg, preferably 100 mg, lycopene in amounts of 8 mg to 100 mg, in particular 5 mg to 40 mg, preferably 10 mg, omega-3 fatty acids, in particular eicosapentaenoic acid and docosahexaenoic acid, each in amounts of 10 mg to 500 mg, in particular 30 mg to 200 mg, preferably 50 to 100 mg, ubiquinone-10 in amounts of 5 mg to 200 mg, in particular 10 mg to 100 mg, preferably 35 mg, are contained in the combination preparation.

5. The combination preparation for use according to at least one of claims 1 to 4, **characterized in that** eicosapentaenoic acid is contained in the combination preparation in amounts of 10 mg to 500 mg, in particular 50 mg to 300 mg, preferably 90 mg, and docosahexaenoic acid is contained in the combination preparation in amounts of 10 mg to 200 mg, in particular 40 mg to 100 mg, preferably 60 mg.

6. The combination preparation for use according to one of claims 1 to 5, **characterized in that** further active ingredients such as magnesium, vitamin K, molybdenum, nicotinamide, chromium, calcium, β-carotene, probiotics, phosphorus, manganese and/or vitamin A are contained.

7. The combination preparation for use according to claim 6, **characterized in that** magnesium and/or a magnesium compound in amounts of 5 mg to 500 mg, in particular 10 mg to 250 mg, preferably 30 mg to 100 mg, vitamin K in amounts of 0.001 mg to 0.5 mg, in particular 0.01 mg to 0.1 mg, preferably 0,05 mg to 0.08 mg, molybdenum in amounts of 0.001 mg to 0.5 mg, in particular 0.01 mg to 0.1 mg, preferably 0.05 mg to 0.08 mg, nicotinamide in amounts of 8 mg to 100 mg, in particular 5 mg to 40 mg, preferably 10 mg, chromium in amounts of 0.001 mg to 0.5 mg, in particular 0.01 mg to 0.1 mg, preferably 0,05 mg to 0.08 mg, calcium in amounts of 25 mg to 400 mg, in particular 50 mg to 200 mg, preferably 100 mg, β-carotene in amounts of 750 µg to 12 mg, in particular 1.5 mg to 6 mg, preferably 3 mg, probiotics in amounts of 100 µg to 10 mg, in particular 200 µg to 3 mg, preferably 1 mg, phosphorus in amounts of 5 mg to 500 mg, in particular 10 mg to 250 mg, preferably 30 mg to 100 mg, manganese in amounts of 100 µg to 5 mg, in particular 200 µg to 3 mg, preferably 1 mg, vitamin A in amounts of 20 µg to 500 µg, in particular 50 µg to 250 µg, preferably 100 µg, are contained in the combination preparation.

8. The combination preparation for use in a method for the treatment of female sub- and infertility associated with endometriosis, comprising at least C vitamin, E vitamins, B1 vitamin, B2 vitamin, B5 vitamin, B6 vitamins, B7 vitamin, B9 vitamin, B12 vitamins, niacin, zinc, iron, selenium, iodine, copper, magnesium, N-acetylcysteine, lycopene, omega-3 fatty acids, ubiquinone-10, D vitamins, **characterized in that** lycopene is contained in the combination preparation in an amount with a lower limit of 8 mg.

9. The combination preparation for use according to claim 8 for use in the treatment of female sub- and infertility associated with endometriosis, **characterized in that** at least 100 mg of C vitamin, 12 mg of E vitamins, 3 mg of B1 vitamin, 3 mg of B2 vitamin, 12 mg of B5 vitamin, 4 mg of B6 vitamins, 100 µg of B7 vitamin, 800 µg of B9 vitamin, 7 µg of B12 vitamins, 35 mg of niacin, 5 mg of zinc, 7.7 mg of iron, 110 µg of selenium, 150 µg of iodine, 1 mg of copper, 30 mg of magnesium, 100 mg of N-acetylcysteine, 10 mg of lycopene, 35 mg of ubiquinone-10 and 20 µg of D vitamins in one administration form and 150 mg omega-3 fatty acids in another administration form constitute the combination preparation as active ingredients.

## Revendications

1. Préparation en combinaison pour une utilisation dans un procédé destiné au traitement de la sous-fertilité et de l'infertilité féminine associées à une endométriose, comprenant au moins les principes actifs vitamine C, vitamines E, vitamine B1, vitamine B2, vitamine B5, vitamines B6, vitamine B7, vitamine B9, vitamines B12, niacine, zinc, fer, sélénium, iode, cuivre, N-acétylcystéine, lycopène, acides gras oméga-3, ubiquinone-10 et éventuellement vitamines D, **caractérisée en ce que** la préparation en combinaison contient du lycopène en une quantité présentant une limite inférieure de 8 mg.

2. Préparation en combinaison pour l'utilisation selon la revendication 1, **caractérisée en ce qu'**elle contient la vitamine C et la N-acétylcystéine selon un rapport de 1:1.

3. Préparation en combinaison pour l'utilisation selon la revendication 1 ou 2, **caractérisée en ce que** les acides gras oméga-3 sont présents selon un rapport de l'acide éicosapentaénoïque (EPA) à l'acide docosahexaénoïque (DHA) de 3:2.

4. Préparation en combinaison pour une utilisation selon au moins l'une des revendications 1 à 3, **caractérisée en ce que** la préparation en combinaison contient la vitamine C en des quantités de 25 mg à 400 mg, en particulier de 50 mg à 200 mg, de préférence de 100 mg, les vitamines E en des quantités de 5 mg à 80 mg, en particulier de 10 mg à 40 mg, de préférence de 12 mg, la vitamine B1 en des quantités de 750 µg à 12 mg, en particulier de 1,5 mg à 6 mg, de préférence de 3 mg, la vitamine B2 en des quantités de 750 µg à 12 mg, en particulier de 1,5 mg à 6 mg, de préférence de 3 mg, la vitamine B5 en des quantités de 3 mg à 50 mg, en particulier de 6 mg à 25 mg, de préférence de 12 mg, la vitamine B6 en des quantités de 1 mg à 20 mg, en particulier de 2 mg à 10 mg, de préférence de 4 mg, les vitamines B7 en des quantités de 50 µg à 1 mg, en particulier de 100 µg à 500 µg, de préférence de 100 µg, la vitamine B9 en des quantités de 100 µg à 5 mg, en particulier de 200 µg à 2 mg, de préférence de 800 µg, les vitamines B12 en des quantités de 1 µg à 50 µg, en particulier de 2 µg à 25 µg, de préférence de 7 µg, éventuellement les vitamines D en des quantités de 1 µg à 50 µg, en particulier de 5 µg à 20 µg, de préférence de 10 µg, la niacine en des quantités de 5 mg à 200 mg, en particulier de 10 mg à 100 mg, de préférence de 35 mg, le zinc et/ou un composé du zinc en des quantités de 1 mg à 20 mg, en particulier de 2 mg à 10 mg, de préférence de 5 mg, le fer et/ou un composé du fer en des quantités de 1 mg à 30 mg, en particulier de 2 mg à 15 mg, de préférence de 7,5 mg, le sélénium et/ou un composé du sélénium en des quantités de 20 µg à 500 µg, en particulier de 50 µg à 250 µg, de préférence de 100 µg, le cuivre et/ou un alliage de cuivre en des quantités de 100 µg à 5 mg, en particulier de 200 µg à 3 mg, de préférence de 1 mg, l'iode et/ou un composé de l'iode en des quantités de 20 µg à 800 µg, en particulier de 50 µg à 400 µg, de préférence de 150 µg, la N-acétylcystéine en des quantités de 25 mg à 400 mg, en particulier de 50 mg à 200 mg, de préférence de 100 mg, le lycopène en des quantités de 8 mg à 100 mg, en particulier de 5 mg à 40 mg, de préférence de 10 mg, les acides oméga-3, en particulier l'acide éicosapentaénoïque et l'acide docosahexaénoïque, chacun en des quantités de 10 mg à 500 mg, en particulier de 30 mg à 200 mg, de préférence de 50 à 100 mg, l'ubiquinone-10 en des quantités de 5 mg à 200 mg, en particulier de 10 mg à 100 mg, de préférence de 35 mg.

5. Préparation en combinaison pour l'utilisation selon au moins l'une des revendications 1 à 4, **caractérisée en ce que** la préparation en combinaison contient l'acide éicosapentaénoïque en des quantités de 10 mg à 500 mg, en particulier de 50 mg à 300 mg, de préférence de 90 mg, et l'acide docosahexaénoïque en des quantités de 10 mg à 200 mg, en particulier de 40 mg à 100 mg, de préférence de 60 mg.

6. Préparation en combinaison pour l'utilisation selon l'une des revendications 1 à 5, **caractérisée en ce qu'**elle contient d'autres principes actifs tels que le magnésium, la vitamine K, le molybdène, le nicotinamide, le chrome, le calcium, le β-carotène, des probiotiques, le phosphore, le manganèse et/ou la vitamine A.

7. Préparation en combinaison pour l'utilisation selon la revendication 6, **caractérisée en ce que** la préparation en combinaison contient le magnésium et/ou un composé du magnésium en des quantités de 5 mg à 500 mg, en particulier de 10 mg à 250 mg, de préférence de 30 mg à 100 mg, la vitamine K en des quantités de 0,001 mg à 0,5 mg, en particulier de 0,01 mg à 0,1 mg, de préférence de 0,05 mg à 0,08 mg, le molybdène en des quantités de 0,001 mg à 0,5 mg, en particulier de 0,01 mg à 0,1 mg, de préférence de 0,05 mg à 0,08 mg, le nicotinamide en des quantités de 8 mg à 100 mg, en particulier de 5 mg à 40 mg, de préférence de 10 mg, le chrome en des quantités de 0,001 mg à 0,5 mg, en particulier de 0,01 mg à 0,1 mg, de préférence de 0,05 mg à 0,08 mg, le calcium en des quantités de 25 mg à 400 mg, en particulier de 50 mg à 200 mg, de préférence de 100 mg, le β-carotène en des quantités de 750 µg à 12 mg, en particulier de 1,5 mg à 6 mg, de préférence de 3 mg, les probiotiques en des quantités de 100 µg à 10 mg, en particulier de 200 µg à 3 mg, de préférence de 1 mg, le phosphore en des quantités de 5 mg à 500 mg, en particulier de 10 mg à 250 mg, de préférence de 30 mg à 100 mg, le manganèse en des quantités de 100 µg à 5 mg, en particulier de 200 µg à 3 mg, de préférence de 1 mg, la vitamine A en des quantités de 20 µg à 500 µg, en particulier de 50 µg à 250 µg, de préférence de 100 µg.

8. Préparation en combinaison pour une utilisation dans un procédé destiné au traitement de la sous-fertilité et de l'infertilité féminine associées à l'endométriose, comprenant au moins de la vitamine C, des vitamines E, de la vitamine B1, de la vitamine B2, de la vitamine B5, des vitamines B6, de la vitamine B7, de la vitamine B9, des vitamines B12, de la niacine, du zinc, du fer, du sélénium, de l'iode, du cuivre, du magnésium, de la N-acétylcystéine, du lycopène, des acides gras oméga-3, de l'ubiquinonc-10, des vitamines D, **caractérisée en ce que** la préparation en combinaison contient le lycopène en une quantité présentant une limite inférieure de 8 mg.

9. Préparation en combinaison pour l'utilisation selon la revendication 8, pour une utilisation dans le traitement de la sous-fertilité et de l'infertilité féminine associées à l'endométriose, **caractérisée en ce qu'**au moins 100 mg de vitamine C, 12 mg de vitamines E, 3 mg de vitamine B1, 3 mg de vitamine B2, 12 mg de vitamine B5, 4 mg de vitamines B6, 100 µg de vitamine B7, 800 µg de vitamine B9, 7 µg de vitamines B12, 35 mg de niacine, 5 mg de zinc, 7,7 mg de fer, 110 µg de sélénium, 150 µg d'iode, 1 mg de cuivre, 30 mg de magnésium, 100 mg de N-acétylcystéine, 10 mg de lycopène, 35 mg d'ubiquinone-10 et 20 µg de vitamines D dans une forme posologique, ainsi que 150 µg d'acides gras oméga-3 dans une autre forme posologique, forment en tant que principes actifs la préparation en combinaison.
